# FASCICULE DE BREVET EUROPEEN

(11) **EP 3 206 734 B1**
(45) Date de publication et mention de la délivrance du brevet: **22.05.2019**
(21) Numéro de dépôt: 15801931.5
(22) Date de dépôt: 15.10.2015
(51) Int. Cl.: A61M 1/28, A61B 50/20, A61M 5/14, A61M 1/16

(54) **POIGNÉE D'UN DISPOSITIF MÉDICAL**
GRIFF EINER MEDIZINISCHEN VORRICHTUNG
HANDLE OF A MEDICAL DEVICE

(30) Priorité: 17.10.2014 EP 14189391
(43) Date de publication de la demande: 23.08.2017
(73) Titulaire: Debiotech S.A., 1004 Lausanne (CH)
(72) Inventeur: KRUMMENACHER, Renaud, 1004 Lausanne (CH); NEFTEL, Frédéric, 1005 Lausanne (CH)
(74) Mandataire: Weihs, Bruno Konrad
(86) Numéro de dépôt international: PCT/IB2015/057923
(87) Numéro de publication internationale: WO 2016/059589

(56) Documents cités:
- WO-A2-01/08722
- US-A- 4 211 380
- US-A1- 2010 270 225

## Description

### Domaine de l'invention

L'invention concerne une poignée d'un dispositif médical qui permet d'appréhender le dispositif médical mais qui est également utilisé en tant que support pour un élément distinct du dispositif médical, par exemple un filtre. La présente demande revendique la priorité de EP 14189391.7, déposée le 17 octobre 2014 au nom de Debiotech, dont le contenu intégral doit être considéré comme faisant partie de la présente demande.

### Etat de la technique

Certains dispositifs médicaux sont pourvus d'une poignée. Cette poignée n'est qu'un élément permettant de transporter ou manipuler le dispositif médical, autrement dit elle n'a pas d'autre fonction que la fonction classique d'une poignée. Or cet élément pratique a un certain coût pour une fonction pratique mais relativement peu fondamentale.

D'un autre côté, certains systèmes tel que les systèmes de dialyse comprennent des éléments fonctionnels pour le traitement, mais ces éléments fonctionnels peuvent être des éléments distincts de l'appareil « principal ». Il s'agit par exemple du filtre d'un système d'hémodialyse qui est maintenu durant le traitement sur l'appareil dans une certaine position (verticale de préférence, pour permettre l'évacuation des bulles). Ainsi durant le traitement ces éléments sont maintenu ensembles. Ces systèmes médicaux sont donc pourvus de support n'ayant que la fonction de maintenir ledit élément contre l'appareil « principal ». Les documents portant les numéros de publication suivant US 4,211,381 A, US 4,211,380, US 2010/270225 A1 et WO 01/08722 A2 exposent des systèmes de support pour filtre mais le support n'est pas une poignée pour un dispositif. Autrement dit, chaque élément n'a qu'une fonction unique, ce qui n'est pas économiquement optimal.

### Description générale de l'invention

L'invention est définie par les revendications.

La présente invention a pour but principal de palier les inconvénients des systèmes connus en proposant de fusionner la fonction de poignée et de support d'un élément. Une synergie est ainsi créée afin de minimiser le nombre de pièces dans un objectif économique et/ou d'optimisation des fonctions, de l'encombrement et/ou de rationaliser l'utilisation du dispositif.

Un système médical peut comprendre au moins un dispositif jetable et/ou au moins un dispositif réutilisable. De manière générale, un dispositif jetable est un dispositif à usage unique et ainsi après son utilisation le dispositif jetable ne peut être réutilisé. Le dispositif jetable est jeté car il comprend au moins un élément (chemin fluidique par exemple) qui est entré en contact avec un fluide qui peut être le sang du patient, une solution médicamenteuse ou autre. La réutilisation d'un tel dispositif nécessiterait des étapes de nettoyage et de stérilisation couteuse et pouvant altérer le dispositif. De plus, ledit élément peut être difficilement séparable ou non dissociable du dispositif et c'est pour cette raison que l'ensemble du dispositif comprenant cet élément est alors considéré comme à usage unique. Par exemple un dispositif jetable peut être un filtre au travers duquel passe un fluide (par exemple dialysat et/ou sang). A contrario, un dispositif réutilisable est un dispositif qui peut être utilisé plusieurs fois. Ainsi, un dispositif réutilisable peut être utilisé successivement avec plusieurs dispositifs jetables et inversement un dispositif jetable ne peut être utilisé qu'avec un seul dispositif réutilisable.

En particulier, dans le domaine de l'hémodialyse, le système médical comprend au moins un dispositif réutilisable (également appelé permanent) qui comprend généralement les éléments les plus sophistiqués ou onéreux (par exemple : l'électronique, les capteurs, les actuateurs,...) et au moins un dispositif jetable. Il peut s'agir notamment de la distribution fluidique (cassette, tube, sac, poche de chauffage,...), du filtre et dans certains cas d'un dispositif sorbent qui permet de filtrer le dialysat utilisé afin de le recycler, par exemple par absorption ou adsorption de certaines substances contenues dans la solution filtré. Dans certain cas, certains de ces éléments peuvent être réutilisés par exemple après nettoyage.

Lors de l'utilisation de tels systèmes médicaux, certains éléments du dispositif jetable peuvent être maintenus par un autre dispositif tel que le dispositif permanent. Par exemple,, le filtre (dispositif jetable) peut être maintenu par l'appareil principal (dispositif permanent) durant le traitement. En guise d'information, un filtre est un dispositif jetable dans lequel circule d'un côté le sang du patient circule et de l'autre un fluide (dialysat) dans le but d'effectuer les échanges nécessaires (urée, eau,...) entre ces deux fluides comme l'effectuerait un rein.

Dans le cadre de l'invention, le support (utilisé par exemple pour fixer le filtre) est un élément de la poignée de transport du dispositif. Autrement dit, la poignée comprend un support permettant de fixer de façon amovible un autre élément tel qu'un filtre. Cet autre élément peut être un dispositif jetable ou réutilisable. Pour rendre la lecture plus facile, il sera utilisé le mot filtre pour désigner cet autre élément et le terme dispositif principal pour désigner le dispositif sur lequel est fixé le support (le dispositif principal peut être ou non l'appareil principal). Le dispositif principal peut être un cycler et/ou comprendre (de façon amovible ou non) un sorbent.

Ainsi ladite poignée à une double fonction, celle de manoeuvrer, tenir ou portée le dispositif (sur lequel elle est fixé) et celle de maintenir le filtre. L'intérêt est majeur, le filtre et son support forment ainsi un élément facilement appréhendable par un utilisateur. Cet ensemble forme la poignée du dispositif. Le dispositif principal et le filtre sont ainsi transportable et/ou manipulable simplement en appréhendant un seul élément (la poignée) tout en assurant la position fonctionnelle du filtre (angle).

L'ensemble support/filtre est adapté pour être facilement appréhendé par la main d'un utilisateur. Toutefois, il est possible que le support sans le filtre soit adapté pour également être appréhendé par la main d'un utilisateur, ainsi la poignée peut être fonctionnelle même sans le filtre.

Dans un mode de réalisation, le dispositif principal est un dispositif jetable. Autrement dit, le filtre est maintenu sur un dispositif jetable. Ainsi, à la fin du traitement l'ensemble filtre et dispositif principale (jetable) est jeté sans avoir à les séparer. Cela permet de faciliter la manipulation de l'ensemble. Le dispositif jetable peut être le sorbent ou un système de distribution fluidique.

Dans un autre mode de réalisation, le dispositif principal est un dispositif réutilisable. Autrement dit, le filtre est maintenu sur un dispositif permanent, tout en permettant la préhension à travers ladite poignée du dispositif permanent.

Un des problèmes relatif à une poignée est qu'elle peut servir à déplacer un dispositif (permanent ou jetable) ayant un certain poids (500g ou plus). Un filtre (de forme plutôt tubulaire et/ou longitudinale) représente en lui-même une poignée idéale de part sa forme. Toutefois, si le filtre est le seul élément de préhension qui forme la poigné du dispositif principale alors il faut que le filtre soit suffisamment solide pour assurer sa fonction de poignée. Ainsi dans un mode de réalisation, la poigné comprend un élément de préhension couplé mécaniquement au support de manière à limiter les contraintes mécanique sur le filtre lorsqu'il est utilisé en tant que poignée. Dans ce cas, le support est adapté pour recevoir et maintenir solidement le filtre sur l'élément de préhension.

Ainsi, une des originalités de l'invention est de se servir de la poignée comme support pour le filtre (avec la possibilité d'une fixation, par exemple au moyen d'un clipsage), tout en assurant la préhension du filtre à l'aide de la main au travers de la poignée elle-même. Ainsi c'est l'élément support de la poignée et non le filtre qui est soumis aux principales forces mécanique (tout en se servant de la forme cylindrique du filtre pour constituer la forme définitive facile à appréhender de la poignée).

### Liste des figures

L'invention sera mieux comprise ci-après au moyen de quelques exemples illustrés.
Il va de soi que l'invention ne se limite pas à ces modes de réalisation.
Figure 1 schématise un exemple de système hémodialyse comprenant une unité de sorbent.
Figure 2 montre le dispositif comprenant une poignée telle que décrite par la présente invention.
Figure 3 illustre l'agencement du filtre sur la poignée.
Figure 4 illustre l'appréhension de l'ensemble par la main de l'utilisateur.
Figure 5 représente un système d'hémodialyse complet.
Figure 6 schématise un mode réalisation où tout ou partie d'un chemin fluidique est compris dans le dispositif principal.
Figure 7 schématise un mode réalisation où la majeur partie du chemin fluidique du dialysat est à l'extérieur du dispositif principal.
Figure 8 est une vue en coupe de l'ensemble poigné/support.

### Références numériques utilisées dans les figures

- 1: Système d'hémodialyse
- 2: Patient
- 3: Appareil principal
- 4: Filtre
- 5: Sorbent
- 6: Circuit du sang
- 7: Circuit du dialysat
- 10: Dispositif principal (i.e. comprenant une poignée tel que décrite par l'invention)
- 11: Support du filtre
- 12: Elément de préhension
- 13, 14: Eléments de liaison mécanique
- 15: Main d'un utilisateur
- 16: Connecteur/Port de connexionfluidique
- 17: Ensemble poigné/support
- 18: Orifice de passage

### Description détaillée de l'invention

Dans le présent document, la description détaillée de l'invention comporte des modes de réalisation de dispositifs, de systèmes et de méthodes présentés à titre d'illustration. Il est bien entendu que d'autres modes de réalisation sont envisageables et peuvent être apportées sans s'écarter de la portée ou l'esprit de l'invention. La description détaillée qui suit, par conséquent, ne doit pas être prise dans un sens limitatif.

Sauf indication contraires, les termes scientifiques et techniques utilisé dans le présent document ont des significations couramment utilisés par l'homme du métier. Les définitions apportées dans ce document sont mentionnées afin de faciliter la compréhension des termes fréquemment utilisés et ne sont pas destinées à limiter la portée de l'invention.

Les indications de direction utilisées dans la description et les revendications, telles que "haut", "bas", "gauche", "droite", "supérieur", "inférieur", et autres directions ou orientations sont mentionnées afin d'apporter plus de clarté en référence aux figures. Ces indications ne sont pas destinées à limiter la portée de l'invention.

Les verbes « avoir », « comprendre », « inclure » ou équivalent sont utilisés dans le présent document dans un sens large et signifie de façon générale « inclut, mais sans s'y limiter.

Le terme "ou" est généralement employé dans un sens large comprenant "et/ou" à moins que le contexte indique clairement le contraire.

Dans ce document, le terme « poignée » doit être compris comme un élément ou un ensemble d'éléments fixé à un dispositif ayant au moins pour fonction substantielle de manoeuvrer, tenir ou porter ledit dispositif et qui est adapté pour être appréhendé par au moins une main d'un utilisateur.

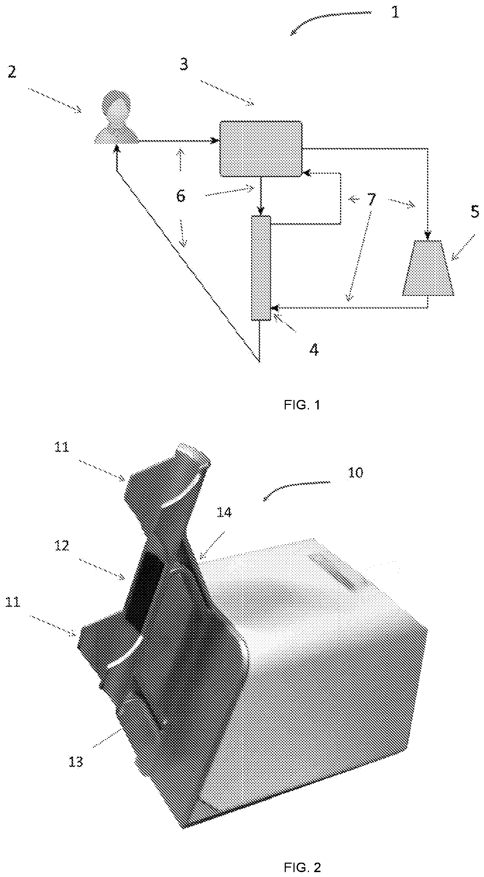

La figure 1 schématise brièvement un exemple de système d'hémodialyse (1). Le système comprend deux circuits distincts (6, 7), le premier est le sang et le deuxième le dialysat. Le sang est prélevé sur le patient grâce à une pompe à sang agencée dans l'appareil principale (le cycler) (3). Le sang va ensuite passer dans le filtre (4) afin d'être purifié puis il retourne dans le patient (2). Le circuit du dialysat (7) peut être différent en fonction des systèmes. Il peut comprendre un sac de dialysat frais qui va passer dans le filtre (4). Ensuite, ce dialysat utilisé peut être stocké dans un sac spécifique. Dans notre exemple, un dispositif de type sorbent (5) est agencé entre l'appareil principal (3) et le filtre (4). Dans d'autres systèmes, le sorbent peut être agencé ailleurs. Ainsi grâce au sorbent, le dialysat utilisé peut être rincé de ses impuretés afin d'être réutilisé pendant le traitement (éventuellement par reconstitution du liquide avec un liquide contenant des solutés concentrés nécessaires à la reconstitution du dialysat à partir, notamment, d'eau). La figure 1 peut également schématiser un exemple de système de dialyse péritonéale avec reconstitution du dialysat. Dans ce cas, le chemin fluidique (6) contient du dialysat qui est injecté dans puis retiré de la cavité péritonéale du patient. Le chemin fluidique (7) permettrait alors dans ce cas de « laver » le dialysat usé qui a été retiré au patient afin qu'il soit réutilisé.

Normalement, tout ce qui a été mouillé par le sang ou le dialysat est à usage unique. Pour éviter de jeter l'appareil principal (le cycler) (3), le système utilise par exemple une cassette (non représentée) qui sert d'interface entre l'appareil principal et le fluide. De cette manière juste la cassette est jetée. La cassette, le filtre (4) et le sorbent (5) sont en communication fluidique à travers le circuit du dialysat (7) et sont donc, en général, destinés également à un usage unique.

Le filtre se présente comme un élément comprenant un corps principal longitudinal et deux extrémités opposés. A ces extrémités ou proche d'elle, des tubes permettent une connexion fluidique au circuit de sang et de dialysat.

La figure 2 illustre un mode de réalisation préférée, le dispositif principal (10) comprend une poignée solidement fixé et/ou lié de façon fixe au dispositif via au moins un moyen de liaison mécanique (13, 14). La poignée peut comprendre un élément de préhension (12) qui peut permettre à la main de l'utilisateur de passer entre la poignée et le dispositif (10). Grâce à cet élément de préhension, la poignée peut être utilisée sans le filtre mais elle permet également d'améliorer sensiblement la résistance mécanique de l'ensemble. La poignée comprend en outre au moins un support adapté pour maintenir de façon amovible le filtre sur l'élément de préhension. Dans un mode de réalisation, un sorbent est disposé à l'intérieur du dispositif principal ou le sorbent est le dispositif principal. Dans ce dernier cas, le sorbent aura des pieds pour être posé sur une surface durant son utilisation (pendant le traitement).

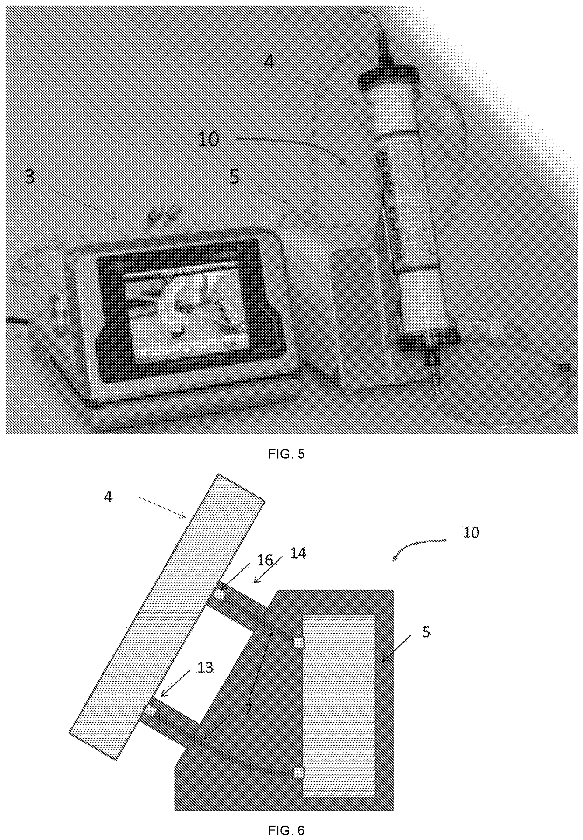

Dans un mode de réalisation divulgué en figure 6, tout ou partie du chemin fluidique (7) reliant le filtre (4) au sorbent (5) peut être agencé dans le dispositif principal et/ou dans les éléments de liaison mécanique (13, 14). Le dispositif principal peut également comprendre des connecteurs fluidiques adaptés pour connecter fluidiquement le filtre et/ou le sorbent au chemin fluidique (6,7). Les chemins fluidiques peuvent être des tubes fixés de façon amovible ou non au dispositif principal et/ou aux éléments de liaison mécanique (13, 14).

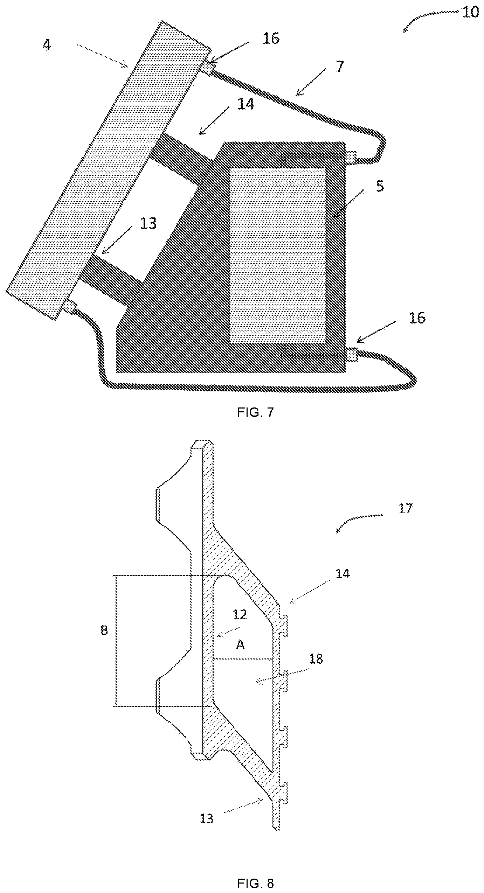

Dans un mode de réalisation divulgué en figure 7, le sorbent (5) et le filtre (4) sont fluidiquement connectés via des tubes et ports de connexion (16). Un système de distribution de fluide, tel que décrit dans la demande international portant le numéro PCT/IB2014/063214 peut être utilisé pour transporter le fluide à travers le chemin fluidique (7). Ces tubes et ports peuvent être agencés à l'extérieur du dispositif.

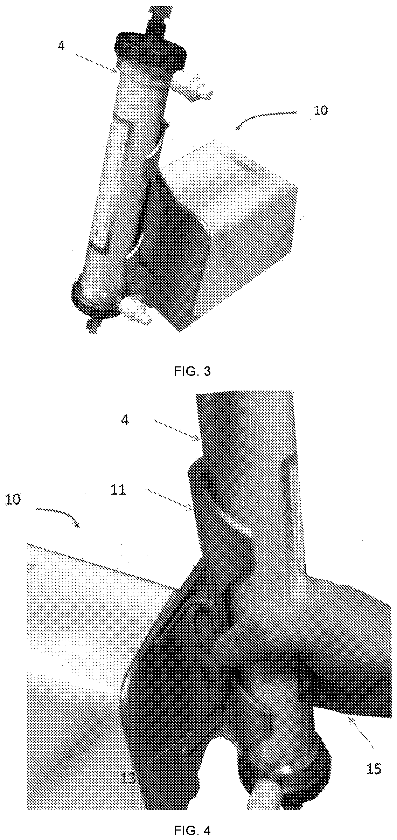

La figure 3 expose l'agencement du filtre (4) sur le dispositif (10). Dans ce mode de réalisation, l'élément de préhension (12) s'étend longitudinalement contre le corps du filtre (4). Le support peut comprendre un ou deux éléments agencés substantiellement aux deux extrémités de l'élément de préhension et ou proche des extrémités du filtre.

La figure 4 illustre la préhension de la poignée par la main d'un utilisateur (15). Ainsi, le filtre (4) et l'élément de préhension (12) forment un ensemble adapté pour être appréhendé facilement par la main d'un utilisateur alors que l'élément de préhension et le filtre sont des éléments totalement distincts. L'existence de cet élément de préhension (12) permet d'éviter de transférer l'essentiel des contraintes mécanique sur le corps du filtre (4) et ne nécessite donc pas une fixation nécessairement sécurisée du filtre sur la poignée. Autrement dit, même lorsque le dispositif est lourd, l'élément de préhension assure une résistance mécanique suffisante pour que le filtre ne subisse pas trop de contraintes qui risqueraient de l'endommager ou pour que l'ensemble ne nécessite pas de fixation sécurisée entre le filtre et la poignée elle-même.

Dans un mode de réalisation divulgué par la figure 5, le système médical complet comprend un appareil principal (3), un filtre (4) maintenu sur un dispositif principal(10) comprenant la poignée. Dans ce mode de réalisation, le dispositif est un dispositif de sorbent (5). Dans ce mode de réalisation l'appareil principal et le dispositif principal sont distincts mais dans un autre mode de réalisation le dispositif principal peut être l'appareil principal.

Dans un mode de réalisation possible, l'élément de préhension (avec ou sans le filtre), le support et ou les éléments de liaison mécanique peuvent être désolidarisés du dispositif principal de sorte qu'ilspuissent être réutilisés avec un autre dispositif principal.

Dans un autre mode de réalisation, un ensemble constitué d'au moins deux éléments distincts forme une poignée d'un dispositif médial. Ce dispositif médical peut être un dispositif jetable ou réutilisable d'un système médical. Ledit ensemble comprend un premier élément et un deuxième élément qui est distinct du dispositif médical et du premier élément. Cet ensemble est adapté pour être saisi par l'utilisateur au moyen d'une main. Le deuxième élément peut être doté d'une forme facilitant la préhension, par exemple de forme cylindrique et/ou longitudinale. Le deuxième élément peut être en outre maintenu de manière réversible au premier élément, ce dernier formant ainsi un élément de support du deuxième. L'ensemble forme ainsi un élément de levage du dispositif médical adapté pour être saisi par une main pour lever/transporter/déplacer le dispositif médical. Le deuxième élément peut en outre être un élément fonctionnel du système médical. Par exemple, il peut être adapté pour effectuer une tache/action nécessaire pour la thérapie d'un patient. L'ensemble étant

La figure 8 expose l'ensemble poigné/support sans le dispositif principal (10) et sans le filtre (4). Cet ensemble peut être attaché de façon amovible au dispositif principal grâce aux éléments de fixation mécanique (13, 14) qui peuvent comprendre des éléments de couplage (par exemple en forme de bouton) qui s'emboitent avec des éléments de couplage adaptés au dispositif principal.

La figure 8 expose également l'orifice de passage (18) à travers lequel l'utilisateur introduit au moins partiellement sa main pour saisir la poigné, par exemple l'élément de préhension (12) (avec ou sans le filtre). La distance A peut être comprise entre 1 et 10 cm, idéalement en 2 et 5 cm, par exemple substantiellement égale à 3 cm. La distance B peut être comprise entre 4 et 30 cm, idéalement en 5 et 15 cm, par exemple substantiellement égale à 8 cm.

Selon un mode de réalisation, la poignée comprend un élément de préhension situé à une distance du dispositif médical permettant le passage des doigts, un élément de liaison mécanique permettant de fixer (par exemple mécaniquement) l'élément de préhension au dispositif médical et un support adapté pour recevoir et fixer (par exemple mécaniquement) un dispositif additionnel sur l'élément de préhension. Ainsi, le dispositif additionnel et l'élément de préhension sont des éléments distincts qui forment un ensemble poignée adapté pour être appréhendé par la main d'un utilisateur. En outre, le dispositif additionnel a une fonction substantiellement thérapeutique. Le additionnel peut être un élément fonctionnelle d'épuration du sang du patient, par exemple un filtre.

Préférentiellement, le dispositif additionnel peut être fixé de façon amovible au support.

De plus, l'élément de préhension peut s'étendre longitudinalement le long du dispositif additionnel. Le support peut comprendre au moins un élément de fixation du dispositif additionnel agencé sur l'élément de préhension ou deux éléments de fixation du dispositif additionnel agencés par exemple substantiellement aux deux extrémités de l'élément de préhension.

L'élément de préhension peut être fixé de façon amovible du dispositif médical. En outre, l'élément de préhension avec ou sans le dispositif additionnel peut être détaché de l'ensemble du dispositif médical pour être placé sur un nouveau dispositif médical.

L'invention décrit également un dispositif médical pouvant comprendre une poignée similaire à celle divulgué dans le document. Selon un mode de réalisation, le dispositif médical est jetable ainsi que le dispositif additionnel suite à une unique utilisation, de manière à ce que l'ensemble dispositif médical et dispositif additionnel puissent être jeté facilement.

De plus, le dispositif médical peut être relié fluidiquement au dispositif additionnel et il peut comprendre un sorbent pour fluide de dialyse. Ainsi, le dispositif médical est un élément d'un système de dialyse péritonéale ou un élément d'un système d'hémodialyse.

Dit autrement, la poignée de l'invention peut comprendre deux parties distinctes. Une première partie qui comprend un dispositif additionnel adapté pour être appréhendé par une main d'un utilisateur et une deuxième partie qui comprend des éléments de liaison mécanique permettant de fixer au dispositif médicale un support destiné à recevoir et fixer de manière amovible ladite première partie. Préférentiellement, les éléments de liaison mécanique sont disposés de manière à créer un orifice de passage à travers lequel un utilisateur peut introduire au moins partiellement une main. En outre le dispositif additionnel peut avoir une fonction substantiellement thérapeutique.

## Revendications

1. Dispositif médical (10) adapté pour recevoir un dispositif additionnel comprenant :
ledit dispositif additionnel (4);
une poignée comprenant :
• un élément de préhension (12) situé à une distance du dispositif médical permettant le passage des doigts,
• un élément de liaison mécanique (13, 14) permettant de fixer l'élément de préhension au dispositif médical, et
• un support (11) adapté pour recevoir et fixer au moins temporairement le dispositif additionnel sur l'élément de préhension;
et
une connexion fluidique (16) avec le dispositif additionnel,
dans lequel le dispositif additionnel et l'élément de préhension sont des éléments distincts qui forment un ensemble poignée adapté pour permettre à une personne de saisir l'ensemble et de déplacer le dispositif médical,
dans lequel le dispositif additionnel a une fonction substantiellement thérapeutique.

2. Dispositif médical selon la revendication 1, dans lequel le dispositif additionnel est un élément fonctionnel d'épuration du sang du patient.

3. Dispositif médical selon l'une des précédentes revendications, dans lequel le dispositif additionnel est un filtre.

4. Dispositif médical selon l'une des précédentes revendications, dans lequel le dispositif additionnel est fixé de façon amovible au support.

5. Dispositif médical selon l'une des précédentes revendications 1 à 3, dans lequel le dispositif additionnel est fixé de façon non-amovible au support.

6. Dispositif médical selon l'une des précédentes revendications, dans lequel l'élément de préhension s'étend longitudinalement le long du dispositif additionnel.

7. Dispositif médical selon l'une des précédentes revendications, dans lequel le support comprend au moins un élément de fixation adapté pour fixer le dispositif additionnel et agencé sur l'élément de préhension.

8. Dispositif médical selon l'une des précédentes revendications, dans lequel le support comprend deux éléments de fixation adaptés pour fixer le dispositif additionnel, et agencés substantiellement aux deux extrémités de l'élément de préhension.

9. Dispositif médical selon l'une des précédentes revendications, dans lequel l'élément de préhension est fixé de façon amovible au dispositif médical.

10. Dispositif médical selon l'une des précédentes revendications, dans lequel l'élément de préhension avec ou sans le dispositif additionnel peut être détaché de l'ensemble du dispositif médical pour être placé sur un nouveau dispositif médical.

11. Dispositif médical selon l'une des précédentes revendications, dans lequel le dispositif médical est jetable ainsi que le dispositif additionnel suite à une unique utilisation, de manière à ce que l'ensemble dispositif médical et dispositif additionnel puisse être jeté facilement.

12. Dispositif médical selon l'une des précédentes revendications, dans lequel ledit dispositif médical est ou comprend un sorbent pour fluide de dialyse.

13. Dispositif médical selon l'une des précédentes revendications, dans lequel le dispositif médical est un élément d'un système de dialyse péritonéale.

14. Dispositif médical selon l'une des précédentes revendications, dans lequel le dispositif médical est un élément d'un système d'hémodialyse.

## Patentansprüche

1. Medizinische Vorrichtung (10), welche dafür ausgelegt ist, eine zusätzliche Vorrichtung aufzunehmen, und umfasst:
die zusätzliche Vorrichtung (4);
einen Griff, welcher umfasst:
• ein Greifelement (12), das sich in einem Abstand von der medizinischen Vorrichtung befindet und das Hindurchgreifen mit den Fingern ermöglicht,
• ein mechanisches Verbindungselement (13, 14), welches ermöglicht, das Greifelement an der medizinischen Vorrichtung zu befestigen, und
• eine Halterung (11), die dafür ausgelegt ist, die zusätzliche Vorrichtung wenigstens zeitweilig am Greifelement aufzunehmen und zu befestigen;
und
eine Fluidverbindung (16) mit der zusätzlichen Vorrichtung,
wobei die zusätzliche Vorrichtung und das Greifelement verschiedene Elemente sind, welche eine Griffeinheit bilden, die dafür ausgelegt ist,
einer Person zu ermöglichen, die medizinische Vorrichtung zu ergreifen und zu bewegen,
wobei die zusätzliche Vorrichtung eine im Wesentlichen therapeutische Funktion hat.

2. Medizinische Vorrichtung nach Anspruch 1, wobei die zusätzliche Vorrichtung ein funktionales Element zur Reinigung des Blutes des Patienten ist.

3. Medizinische Vorrichtung nach einem der vorhergehenden Ansprüche, wobei die zusätzliche Vorrichtung ein Filter ist.

4. Medizinische Vorrichtung nach einem der vorhergehenden Ansprüche, wobei die zusätzliche Vorrichtung lösbar an der Halterung befestigt ist.

5. Medizinische Vorrichtung nach einem der Ansprüche 1 bis 3, wobei die zusätzliche Vorrichtung unlösbar an der Halterung befestigt ist.

6. Medizinische Vorrichtung nach einem der vorhergehenden Ansprüche, wobei sich das Greifelement in Längsrichtung entlang der zusätzlichen Vorrichtung erstreckt.

7. Medizinische Vorrichtung nach einem der vorhergehenden Ansprüche, wobei die Halterung wenigstens ein Befestigungselement umfasst, das dafür ausgelegt ist, die zusätzliche Vorrichtung zu befestigen, und am Greifelement angeordnet ist.

8. Medizinische Vorrichtung nach einem der vorhergehenden Ansprüche, wobei die Halterung zwei Befestigungselemente umfasst, die dafür ausgelegt sind, die zusätzliche Vorrichtung zu befestigen, und im Wesentlichen an den beiden Enden des Greifelements angeordnet sind.

9. Medizinische Vorrichtung nach einem der vorhergehenden Ansprüche, wobei das Greifelement lösbar an der medizinischen Vorrichtung befestigt ist.

10. Medizinische Vorrichtung nach einem der vorhergehenden Ansprüche, wobei das Greifelement mit der zusätzlichen Vorrichtung oder ohne sie von der Gesamtheit der medizinischen Vorrichtung abgetrennt werden kann, um an einer neuen medizinischen Vorrichtung angebracht zu werden.

11. Medizinische Vorrichtung nach einem der vorhergehenden Ansprüche, wobei das Greifelement ebenso wie die zusätzliche Vorrichtung im Anschluss an eine einmalige Verwendung wegwerfbar ist, so dass die aus medizinischer Vorrichtung und zusätzlicher Vorrichtung bestehende Anordnung leicht entsorgt werden kann.

12. Medizinische Vorrichtung nach einem der vorhergehenden Ansprüche, wobei die medizinische Vorrichtung ein Sorptionsmittel für Dialyseflüssigkeit ist oder umfasst.

13. Medizinische Vorrichtung nach einem der vorhergehenden Ansprüche, wobei die medizinische Vorrichtung ein Element eines Peritonealdialysesystems ist.

14. Medizinische Vorrichtung nach einem der vorhergehenden Ansprüche, wobei die medizinische Vorrichtung ein Element eines Hämodialysesystems ist.

## Claims

1. Medical device (10) suited to receiving an additional device, comprising:
said additional device (4);
a handle comprising:
• an element (12) for grasping which is situated at a distance from the medical device that allows for the passage of the fingers,
• a mechanical-connection element (13, 14) that allows the element for grasping to be fixed to the medical device, and
• a support (11) suited to receiving and at least temporarily fixing the additional device on the element for grasping;
and
a fluidic connection (16) to the additional device,
in which the additional device and the element for grasping are distinct elements which form a handle assembly designed to allow a person to grasp the assembly and to move the medical device,
in which the additional device has a substantially therapeutic function.

2. Medical device according to Claim 1, in which the additional device is a functional element for purifying the patient's blood.

3. Medical device according to one of the preceding claims, in which the additional device is a filter.

4. Medical device according to one of the preceding claims, in which the additional device is fixed removably to the support.

5. Medical device according to one of the preceding Claims 1 to 3, in which the additional device is fixed non-removably to the support.

6. Medical device according to one of the preceding claims, in which the element for grasping extends longitudinally along the additional device.

7. Medical device according to one of the preceding claims, in which the support comprises at least one fixing element suited to fixing the additional device and arranged on the element for grasping.

8. Medical device according to one of the preceding claims, in which the support comprises two fixing elements suited to fixing the additional device and arranged substantially at the two ends of the element for grasping.

9. Medical device according to one of the preceding claims, in which the element for grasping is fixed removably to the medical device.

10. Medical device according to one of the preceding claims, in which the element for grasping with or without the additional device can be detached from the assembly of the medical device to be placed on a new medical device.

11. Medical device according to one of the preceding claims, in which the medical device is disposable as is the additional device following a single use, so that the assembly comprising medical device and additional device can be disposed of easily.

12. Medical device according to one of the preceding claims, in which said medical device is or comprises a sorbent for dialysis fluid.

13. Medical device according to one of the preceding claims, in which the medical device is an element of a peritoneal dialysis system.

14. Medical device according to one of the preceding claims, in which the medical device is an element of a hemodialysis system.
